Europäisches Patentamt

(19) European Patent Office  (11) Publication number: **0 060 317**

Office européen des brevets  **B1**

(12) **EUROPEAN PATENT SPECIFICATION**

(45) Date of publication of patent specification: **22.05.85**  (51) Int. Cl.⁴: **B 01 J 37/04,** B 01 J 35/02,
 B 01 J 21/18, B 01 J 27/10,
(21) Application number: **81106730.5**  C 07 C 17/156

(22) Date of filing: **28.08.81**

(54) Use of graphite particles as diluent in a fixed bed exothermic reaction and process for the catalytic oxychlorination of ethylene.

(30) Priority: **18.02.81 JP 23574/81**

(43) Date of publication of application:
 **22.09.82 Bulletin 82/38**

(45) Publication of the grant of the patent:
 **22.05.85 Bulletin 85/21**

(84) Designated Contracting States:
 **BE DE NL**

(56) References cited:
 **FR-A- 510 948**
 **FR-A-2 132 795**
 **FR-A-2 231 621**
 **FR-A-2 390 381**
 **GB-A- 792 706**
 **GB-A-1 373 351**

(73) Proprietor: **KANEGAFUCHI KAGAKU KOGYO
 KABUSHIKI KAISHA
 2-4 Nakanoshima 3-chome
 Kita-ku Osaka-shi Osaka-fu (JP)**

(72) Inventor: **Shiozaki, Ken
 33-banch, 2-chome, Nozoejo Harima-cho
 Kako-gun Hyogo-ken (JP)**
 Inventor: **Ohnishi, Akira
 574-29, Shinobe Befu-cho
 Kakogawa-shi Hyogo-ken (JP)**

(74) Representative: **Türk, Dietmar, Dr. rer. nat. et al
 Redies, Redies, Türk & Gille Patentanwälte
 Brucknerstrasse 20
 D-4000 Düsseldorf 13 (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 060 317 B1

## Description

### Background of the invention
#### 1. Field of the invention

The present invention relates to the use of a novel diluent for a solid catalyst for a fixed bed reactor. More specifically, it relates to the use of an improved catalyst diluent and to a process for the oxychlorination of ethylene in a high yield while preventing locally overheating parts of a catalyst bed.

#### 2. Description of the prior art

In practicing a reaction such as an oxychlorination reaction of ethylene, accompanying huge heat generation, one of the problems to be given care to is control of the reaction temperature. Stated generally, the thermal conductivity of a catalyst per se is low and thus inviting local formation of overheating portion in a catalyst bed. The local formation of overheating portion inevitably brings about a danger such as runaway of reaction, explosion and the like, and even when such a danger is prevented, it increases undesired side reactions such as decomposition to thereby lower selectivity of the desired product. Still worse, the formation of local overheating portion promotes disadvantageously corrosion of the reactor as well as reduction in catalytic performance.

In order to eliminate the foregoing defects in effecting exothermic reactions, there is proposed a process for diluting a catalyst with inert substance particles. As the inert substance particles used for this purpose, silica, alumina, glass beads, graphite, silicon carbide and the like are suggested.

Notwithstanding, when silica, alumina and glass beads are used, the mixing ratio to the catalyst has to be increased to prevent the generation of local overheating portion since the thermal conductivity of these substances is not so high as compared with that of activated alumina, silica alumina and the like widely used as a catalyst and a carrier. As a result, productivity per unit volume of a reactor is forced to be reduced. Moreover, those normally possess approximately twice the apparent specific gravity of the catalyst particles and hence a heterogeneous mixture tends to result when a catalyst is mingled with those diluents. As far as silicon carbide and graphite are concerned, it is pointed out that they cause chemical reaction and degradation in catalytic activity, while exhibiting superior thermal conductivity (Japanese Patent Examined Publication No. 7948/1966). The present inventors have carried out an oxychlorination reaction of ethylene using those diluents commercially available and ascertained that ethyl chloride was prepared in a greater amount than the case where α-alumina was employed as a diluent, and that no difference was observed, as compared with α-alumina, in an effect of controlling reaction temperature. Moreover, the diluents used heretofore have, as a whole, neither substantially the same size nor the same shape, which also causes a heterogeneous mixture.

Another process is proposed to solve the foregoing drawbacks in which the reaction temperature and local overheating is controlled by a combination of several kinds of catalysts having different catalytic activities, without using a diluent. The process, however, is also disadvantageous in that the thermal conductivity of the catalyst particles is so low that it is rather difficult to control overheating portion as compared with the case where a diluent is used.

A series of studies have been made by the present inventors as to a catalyst diluent which not only eliminates the foregoing drawbacks of those known diluents including formation of local overheating portion and a decrease in selectivity as well as catalytic performance, but is capable of enhancing the mixing ratio of a catalyst to a diluent, and the inventors have discovered that only graphite particles in a columnar shape having the specified dimensions achieve these objects.

### Summary of the invention

It is therefore an object of the present invention to provide the possibility to use a catalyst diluent generating no formation of local overheating portion in the catalytic fixed bed.

It is another object of the present invention to provide the possibility to use a catalytic diluent which is capable of increasing the mixing ratio of a catalyst to the diluent and providing a uniform mixture with the catalyst.

It is a further object of the present invention to provide the possibility to use a catalyst diluent exhibiting a superior selectivity and preventing degradation in catalytic performance.

These and other objects of the present invention together with the advantages thereof will become apparent to those skilled in the art from the detailed disclosure of the present invention as set forth hereinbelow.

The present invention encompasses the use of graphite particles as catalyst diluent in a fixed bed exothermic reaction, characterized in that the graphite is in a columnar shape having a diameter of a circular portion of between 3 and 6 mm and a height of between 3 and 6 mm, or having dimensions within the foregoing ranges in a substantially predominant quantity.

Further the invention is concerned with a process for the catalytic oxychlorination of ethylene in a fixed bed exothermic reaction, which is characterized in that the above defined graphite is used as a catalyst diluent, and that the catalyst is used in a cylindrical shape in a substantially predominant quantity.

### Detailed description of the invention

The diluent used according to the present invention may most preferably be in a columnar shape having a diameter of a circular portion of between 4 to 5.5 mm and a height of between 4 to 5.5 mm, or having dimensions within the foregoing ranges in a substantially predominant quantity. In cases where the diameter is more than 6 mm, the thermal conductivity, when mixed with catalyst particles, becomes inferior and uniform and intimate mixing with the catalyst particles is not feasibly provided. Inversely, in cases where the outer diameter is less than 3 mm, there are brought about problems such as increased pressure loss exerted on the effluent and increased cost in manufacture. The diluent used according to the present invention is not necessarily required to possess a complete circle or a circle close thereto as a circular portion of a columnar shape, an oval shape is also within the present invention which provides a satisfactory result. Although it has been known to be able to employ graphite particles as a catalyst diluent, the graphite particles of an irregular shape such as a broken shape or of a great porosity having an apparent specific gravity of less than 1.5 have been used, and thus superior thermal conductivity a graphite per se possesses has not been made the most of. In addition, they exhibit undesired reactivity including an increase in side reactions and hence they are rarely used today. According to experimental results made by the present inventors, they only showed almost the same temperature controlling effect as the other diluents such as α-alumina and even spherical graphite particles also demonstrated similar results. Inversely, the graphite particles having a specified shape of the present invention have been found to produce an outstanding effect of controlling temperature in a catalytic bed as a diluent, thus the present invention being completed.

Another prominent advantage of the diluent used according to the present invention is to increase the mixing ratio of a catalyst to the quantity of the diluent, so that degradation in catalytic activity is not only reduced relatively, but productivity per unit volume of a reactor is raised.

A further advantage of the diluent used according to the present invention is to prevent the occurrence of local overheating portion owing to the irregularity of mixing, because it possesses an apparent specific gravity close to that of catalyst particles commonly employed to thereby facilitate uniform and intimate mixing. On the other hand, α-alumina, fused silica and silicon carbide possess a fairly large apparent specific gravity as compared with a catalyst supported on activated alumina as a carrier and, in consequence, uniform and intimate mixing with a catalyst is not so easy. The heterogeneous mixing also leads to a disadvantage that local overheating portion is likely to take place.

The material of the diluent used according to the present invention is a graphite, which is generally produced by mixing tar and pitch with e.g. petroleum coke, pitch coke and carbon black, molding the mixture, heating it at first at a temperature between 1,000 and 1,300°C and thereafter sintering at a temperature between 2,000 and 3,000°C. A columnar shaped graphite used according to the present invention may be obtained by molding the mixture by a compression type pelletizer after mixing and prior to sintering or by extruding the mixture into a rod shaped article, then cutting it after heating or sintering to be a desired height.

The diluent used according to the present invention need not be a pure single crystal graphite but may preferably be one having a specific gravity of 2.0 or more and an apparent specific gravity of 1.5 or more to enhance a temperature controlling effect as a diluent.

As regards undesired reactivity and degradation in catalytic activity which were deficiencies in the case where the conventional graphite particles were used as a catalyst diluent, the present inventors have discovered after an extensive study that these deficiences are caused by iron remained or entered during the manufacturing, and that the foregoing deficiences can be eliminated by reducing the amount of iron contained in or deposited to the graphite particles to 0.1 percent by weight, more preferably 0.05 percent by weight. The graphite particles containing of iron in an amount of 0.1 percent by weight or less are obtained by use of materials with a low content of iron such as petroleum coke, pitch coke, carbon black and tar pitch and further by preventing iron from mixing into or depositing on the graphite particles from the outside. The graphite particles with a low content of iron may also be prepared by washing out iron contained or deposited with e.g. hydrochloric acid.

When the diluent used according to the present invention is employed on an industrial scale, the diameter of catalyst particles should preferably be close to that of the diluent but is not specifically limited thereto. The catalyst may be in any shape such as sphere, column, ring, singly or in conjunction with two or more. Above all, the most preferable as a catalyst is a cylindrical shape the present inventors made a patent application earlier published (Japanese Patent Application No. 56141842) and when the cylindrical shaped catalyst is used in conjunction with the present diluent, the characteristics the both possess inherently are markedly enhanced to produce unexpected synergistic effects. Furthermore, the catalyst mixed with the diluent used according to the present invention is not limited to one sort, but used in combination with one or more sorts of catalysts which are different in composition, activity and shape. The foregoing applications are also included in the scope of the present invention.

As the reaction that marked effects can be expected by effecting a contact reaction in a fixed bed containing the present diluent on an industrial scale, there are included, by example, an oxychlorination reaction, a vapor phase chlorination, a vapor phase hydrogenation and a vapor phase oxidation.

Hereinafter the present invention will be explained in more detail by examples and comparative examples relating to an oxychlorination reaction producing ethane dichloride.

Examples 1 to 6, Comparative Examples 1 to 5 A reactor used for reaction was a vertical type reactor of a nickel tube having an interior diameter of 26.3 mm and a length of 1,200 mm to the entirety of which a jacket was welded, through which a heating medium (Dowtherm E, trademark of Dow Chemical Japan Co.) was permitted to be recycled in a state of liquid. At an inlet and an outlet of the reactor, were manometers provided to measure the flow resistance through the packed layer of a catalyst and a diluent. The reactant gases were introduced from the upper portion of the reactor. The gases reacted were led from the lower portion of the reactor to the outside of the reactor, cooled at two stages, i.e., first to 5°C and second to −35°C to condense condensable reaction products, then the resultant condensates and non-condensed gases being subjected to the analysis by a gaschromatography in a normal manner.

As a catalyst, a commercially available spherical catalyst made up of a system of $CuCl_2$-$KCl$-$\gamma Al_2O_3$ (20 percent of $CuCl_2$ and 2.3 percent of KCl are contained and the diameter is 5 to 6 mm, hereinafter referred to as "Catalyst A") or a ring-shaped catalyst made up of a system of $CuCl_2$-$KCl$-$\gamma Al_2O_3$ (18 percent of $CuCl_2$ and 1.5 percent of KCl are contained and the height is 5 mm, the outer diameter is 5 mm and the inner diameter is 2.2 mm, hereinafter referred to as "Catalyst B") prepared by the inventors was employed.

The foregoing catalysts were respectively mixed adequately with a variety of diluents to prepare an intimate mixture and 205 ml of the mixture were packed into the upper portion of the reactor and 205 ml of the non-diluted catalyst were packed into the lower portion of the reactor, then the reaction was allowed to take place. The reactant gases were introduced into the reactor, respectively, at a rate of 40 Nl/hr for hydrogen chloride, 21.6 Nl/hr for ethylene and 57 Nl/hr for air. The pressure at the outlet of the reactor was maintained at atmospheric pressure. The reaction temperature was controlled by the adjustment of the temperature of a heating medium in the jacket so that conversion of hydrogen chloride introduced might come to 99 percent. The obtained results are given in Table 1.

TABLE 1

| | Ex. 1 | Ex. 2 | Ex. 3 | Ex. 4 | Ex. 5 | Ex. 6 |
|---|---|---|---|---|---|---|
| Catalyst diluent | | | | | | |
| Sort | Graphite | Graphite | Graphite | Graphite | Graphite | Graphite |
| Shape | Column | Column | Column | Column | Column | Column |
| Diameter (mm) | 5 | 6 | 4 | 5 | 5 | 5 |
| Height (mm) | 5 | 6 | 4 | 5 | 5 | 5 |
| Content of iron (wt%) | 0.03 | 0.02 | 0.03 | 0.02 | 0.2 | 0.04 |
| Apparent specific gravity | 1.6 | 1.6 | 1.6 | 1.6 | 1.6 | 1.45 |
| Catalyst | A | A | A | B | A | A |
| Heat medium temp. $(T_1)$ (°C) | 205 | 202 | 207 | 201 | 204 | 200 |
| Highest reaction temp. $(T_2)$ (°C) | 285 | 288 | 284 | 270 | 285 | 290 |
| $T_2 - T_1$ (°C) | 80 | 86 | 77 | 69 | 81 | 90 |
| Selectivity (%) | | | | | | |
| 1,2-Dichloroethane | 97.4 | 96.9 | 97.6 | 98.7 | 96.6 | 96.6 |
| Ethyl chloride | 0.4 | 0.5 | 0.4 | 0.2 | 0.8 | 0.5 |
| Other halides | 0.9 | 1.0 | 0.8 | 0.5 | 1.1 | 1.2 |
| Combustion rate of ethylene (%) | 1.3 | 1.6 | 1.2 | 0.6 | 1.5 | 1.7 |
| Conversion of HCl (%) | | | | | | |
| Initial stage of reaction | 99.0 | 99.1 | 99.1 | 99.2 | 98.8 | 99.0 |
| After 500-hour reaction | 98.7 | 98.7 | 98.7 | 98.8 | 98.3 | 98.4 |

5

TABLE 1 (cont.).

| | Comp. Ex. 1 | Comp. Ex. 2 | Comp. Ex. 3 | Comp. Ex. 4 | Comp. Ex. 5 |
|---|---|---|---|---|---|
| Catalyst diluent | | | | | |
| Sort | Graphite | Graphite | Graphite | α-alumina | Silicon carbide |
| Shape | Sphere | Broken shape | Column | Sphere | Sphere |
| Diameter (mm) | 4—6 | 3—7 | 7 | 5—6 | 4—6 |
| Height (mm) | — | — | 7 | — | — |
| Content of iron (wt%) | 0.03 | 0.08 | 0.03 | 0.02 | 0.10 |
| Apparent specific gravity | 1.6 | 1.45 | 1.6 | 3.4 | 2.2 |
| Catalyst | A | A | A | A | A |
| Heat medium temp. ($T_1$) (°C) | 195 | 196 | 198 | 192 | 190 |
| Highest reaction temp. ($T_2$) (°C) | 300 | 298 | 277 | 303 | 305 |
| $T_2-T_1$ | 105 | 102 | 99 | 111 | 115 |
| Selectivity (%) | | | | | |
| 1,2-Dichloroethane | 95.7 | 95.9 | 96.1 | 95.1 | 94.7 |
| Ethyl chloride | 0.7 | 0.8 | 0.7 | 0.8 | 0.9 |
| Other halides | 1.4 | 1.3 | 1.3 | 1.6 | 1.8 |
| Combustion rate of ethylene (%) | 2.2 | 2.0 | 1.9 | 2.5 | 2.6 |
| Conversion of HCl (%) | | | | | |
| Initial stage of reaction | 99.2 | 98.9 | 99.0 | 99.1 | 99.0 |
| After 500-hour reaction | 98.1 | 98.0 | 98.2 | 97.8 | 97.5 |

The highest temperature portion (hot spot) was found to be present in the upper portion of the reactor, i.e., in the portion where the diluent was mixed in every example and comparative example.

It is understood from the results of Table 1 that the diluent used according to the present invention is superior in thermal conductivity and reaction results, as compared with graphite particles in other shapes, and α-alumina and silicon carbide commonly employed.

**Claims**

1. Use of graphite particles as catalyst diluent in a fixed bed exothermic reaction, characterized in that the graphite is in a columnar shape having a diameter of a circular portion of between 3 and 6 mm and a height of between 3 and 6 mm.

2. Use according to Claim 1, characterized in that iron is deposited on or contained in the graphite in an amount of 0.1 percent or less.

3. Use of graphite particles as catalyst diluent in a fixed bed exothermic reaction, characterized in that the graphite is in a substantially predominant quantity in a columnar shape having a diameter of a circular portion of between 3 and 6 mm and a height of between 3 and 6 mm.

4. Use according to Claims 1 and 3, characterized in that the apparent specific gravity of the graphite is 1.5 or more.

5. Use according to Claim 3, characterized in that iron is deposited on or contained in the graphite in an amount of 0.1 percent or less.

6. Process for the catalytic oxychlorination of ethylene in a fixed bed exothermic reaction, characterized in that graphite in a columnar shape, having a diameter of a circular portion of between 3 and 6 mm and a height of between 3 and 6 mm, is used as a catalyst diluent and that the catalyst is used in a cylindrical shape in a substantially predominant quantity.

7. Process for the catalytic oxychlorination of ethylene in a fixed bed exothermic reaction, characterized in that graphite in a columnar shape, having a diameter of a circular portion of between 3 and 6 mm and a height of between 3 and 6 mm, is used as a catalyst diluent in a substantially predominant quantity, and that the catalyst is used in a cylindrical shape in a substantially predominant quantity.

**Patentansprüche**

1. Verwendung von Graphitteilchen als Katalysatorverdünnungsmittel in einer exothermen Festbettreaktion, dadurch gekennzeichnet, daß der Graphit in einer säulenartigen Form mit einem Durchmesser des kreisförmigen Teils von zwischen 3 und 6 mm und einer Höhen von zwischen 3 und 6 mm vorliegt.

2. Verwendung nach Anspruch 1, dadurch gekennzeichnet, daß Eisen in einer Menge von 0,1 Prozent oder weniger abgelagert ist auf oder enthalten ist in dem Graphit.

3. Verwendung von Graphitteilchen als Katalysatorverdünnungsmittel in einer exothermen Festbettreaktion, dadurch gekennzeichnet, daß der Graphit in einer wesentlich vorwiegenden Menge in säulenartiger Form mit einem Durchmesser des kreisförmigen Teils von zwischen 3 und 6 mm und einer Höhe von zwischen 3 und 6 mm vorliegt.

4. Verwendung nach den Ansprüchen 1 und 3, dadurch gekennzeichnet, daß das scheinbare spezifische Gewicht des Graphits 1,5 oder mehr ist.

5. Verwendung nach Anspruch 3, dadurch gekennzeichnet, daß Eisen in einer Menge von 0,1 Prozent oder weniger abgelagert ist auf oder enthalten ist in dem Graphit.

6. Verfahren zur katalytischen Oxychlorierung von Ethylen in einer exothermen Festbettreaktion, dadurch gekennzeichnet, daß Graphit in säulenartiger Form mit einem Durchmesser des kreisförmigen Teils von zwischen 3 und 6 mm und einer Höhe von zwischen 3 und 6 mm als ein Katalysatorverdünnungsmittel verwendet wird und daß der Katalysator in einer wesentlich vorwiegenden Menge in zylindrischer Form verwendet wird.

7. Verfahren zur katalytischen Oxychlorierung von Ethylen in einer exothermen Festbettreaktion, dadurch gekennzeichnet, daß Graphit in einer säulenartigen Form mit einem Durchmesser des kreisförmigen Teils von zwischen 3 und 6 mm und einer Höhe von zwischen 3 und 6 mm als ein Katalysatorverdünnungsmittel in einer wesentlich vorwiegenden Menge verwendet wird und daß der Katalysator in einer wesentlich vorwiegenden Menge in einer zylindrischen Form verwendet wird.

**Revendications**

1. L'utilisation de particules de graphite en tant que diluant de catalyseur dans une réaction exothermique sur lit immobilisé, caractérisée en ce que le graphite est doté d'une forme en colonne, dont le diamètre d'une section circulaire se situe entre 3 et 6 mm et la hauteur entre 3 et 6 mm.

2. L'utilisation selon la revendication 1, caractérisée en ce que le fer déposé ou contenu dans le graphite ne dépasse pas une teneur de 0,1 pour cent.

3. L'utilisation de particules de graphite en tant que diluant de catalyseur dans une réaction exothermique sur lit immobilisé, caractérisée en ce que les particules de graphite sont, en grande majorité, dotées d'une formeen colonne dont le diamètre d'une section circulaire se situe entre 3 et 6 mm et la hauteur entre 3 et 6 mm.

4. L'utilisation selon les revendications 1 et 3, caractérisée en ce que le poids spécifique apparent du graphite s'élève à 1,5 ou plus.

5. L'utilisation selon la revendication 3, caractérisée en ce que le fer déposé ou contenu dans le graphite ne dépasse pas une teneur de 0,1 pour cent.

6. Un procédé d'oxychloruration catalytique d'éthylène en réaction exothermique sur lit immobilisé, caractérisé par l'utilisation, en tant que diluant de catalyseur, de graphite en forme de colonne, dont le diamètre d'une section circulaire se situe entre 3 et 6 mm et la hauteur entre 3 et 6 mm, et par la mise en oeuvre de particules de catalyseur en grande majorité de forme cylindrique.

7. Un procédé d'oxychloruration catalytique d'éthylène en réaction exothermique sur lit immobilisé, caractérisé par l'utilisation en tant que diluant de catalyseur de graphite en grande majorité en forme de colonne, dont le diamètre d'une section circulaire se situe entre 3 et 6 mm et la hauteur entre 3 et 6 mm, et par la mise en oeuvre de particules de catalyseur en grande majorité de forme cylindrique.